# EUROPEAN PATENT SPECIFICATION

(11) **EP 1 274 484 B1**
(45) Date of publication and mention of the grant of the patent: **22.09.2004**
(21) Application number: 01933854.0
(22) Date of filing: 19.04.2001
(51) Int. Cl.: A61N 5/06

(54) **THERAPEUTICAL DEVICE FOR EMITTING ELECTROMAGNETIC VISIBLE WAVES**
THERAPEUTISCHE VORRICHTUNG ZUM AUSSENDEN SICHTBARER ELEKTROMAGNETISCHER WELLEN
DISPOSITIF THERAPEUTIQUE PERMETTANT D'EMETTRE DES ONDES ELECTROMAGNETIQUES VISIBLES

(30) Priority: 21.04.2000 IT MI000906
(43) Date of publication of application: 15.01.2003
(73) Proprietor: Boffa, Davide, 20069 Bettola di Pozzo d'Adda (IT); Boffa, Cosimo, 20069 Bettola di Pozzo d'Adda (IT)
(72) Inventor: Boffa, Davide, 20069 Bettola di Pozzo d'Adda (IT); Boffa, Cosimo, 20069 Bettola di Pozzo d'Adda (IT)
(74) Representative: Cattaneo, Elisabetta
(86) International application number: PCT/EP2001/004455
(87) International publication number: WO 2001/080950

(56) References cited:
- WO-A-95/19808
- WO-A-95/26217
- DE-A- 4 143 168
- DE-U- 29 600 470

## Description

The present invention relates to a device for emitting electromagnetic visible waves, more particularly fixed or intermittent lights for the purpose of restoration of some physiological functions of the human body.

More particularly some disorders of the neuro glandular endocrine system cause conditions of ailment that are often treated with drugs removing more the effects than the causes of said illnesses.

In order to avoid these problems some devices are known that use the physiological reactions of the organism to suitable light stimuli and redress the electric, chemical and mechanical activities of the body allowing mitigation or disappearance of the above mentioned pathological condition.

These devices are applied at different points of the body, preferably on the aching areas and have a synchronous action with each other.

Although these devices solve the mentioned technical problem, they have however some drawbacks.

The known devices avoid the contraindications of the drugs, but they are almost always applied in a number greater than one, because they are placed at all the points requiring a redress of the physiological conditions; a complex synchronization of the devices is therefore required for the kind and the timing of the irradiated light beams.

Among these prior art devices, the document WO95/26217 describes an apparatus for colour therapy comprising a diffuser of coloured light. This diffuser is coupled to a feed and control unit, which provides coloured light intensity regulation. According to WO95/26217 the number and size of the panels, the number and arrangement of the light sources are variable depending on the broadness of the area which is to be irradiated and the kind of therapy.

Another device is described in the International patent application WO95/19808. Such a device is for hair-care and is provided with semiconductor diodes emitting light in infrared field (600-1200nm) and in the blue light (350-450nm) for the stimulation of the mitochondria cells. It can be applied as a hood or it can be used as an hand-held drier.

Furthermore use aimed for instance at single aching points, does not easily attain a global redress of the entire glandular endocrine system and therefore of the altered physiological functions, even with the possibility of balancing some functions but unbalancing others.

The object of the present invention is to remove the drawbacks belonging to the prior art devices.

Therefore object of the present invention is a device for emitting electromagnetic visible waves of easy and ready use and limited price, allowing redress of the electric, chemical and mechanical functions of the human being by the application at one single point, regulating the epiphysis pace so as to gradually balance the entire neuro glandular endocrine system until a correct smoothing of all the altered functions is obtained.

Briefly the present invention provides for a device for emitting electromagnetic visible waves comprising an external support operatively associated to electric and electronic means to set the switching state of a plurality of emitters of said waves, wherein some emitters are arranged on a single plane of said support, while other emitters are arranged close to the summit of said support.

The device for emitting electromagnetic visible waves according to the invention is characterized by the features recited in claim 1. Further advantageous features of the device are recited in the dependent claims.

Other advantages, details and features of the device for emitting electromagnetic visible waves according to the invention will be better understood by reading the following description making reference to the accompanying drawings in which a preferred embodiment is shown as an illustrative but nonlimiting example.

Fig. 1 is a schematic view in horizontal projection of the device for emitting electromagnetic visible waves of the invention.

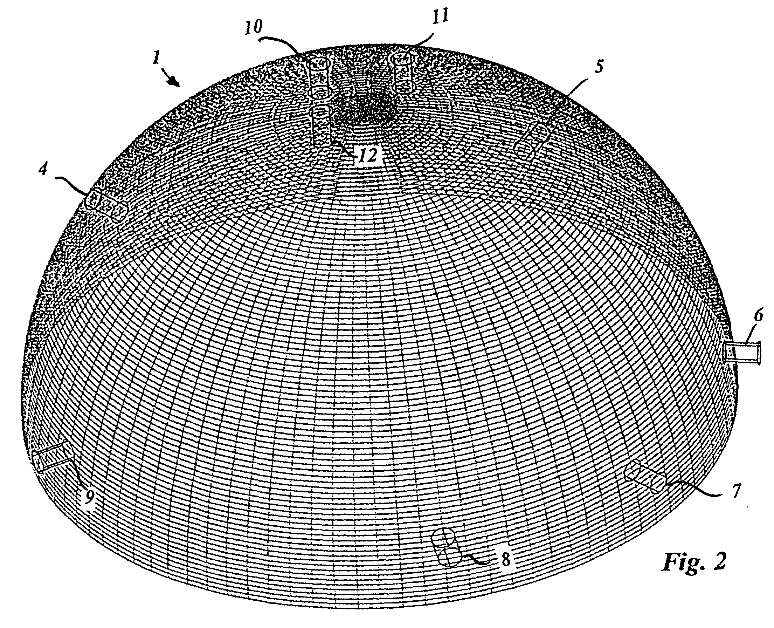

Fig. 2 is a schematic perspective view of the device shown in Fig. 1.

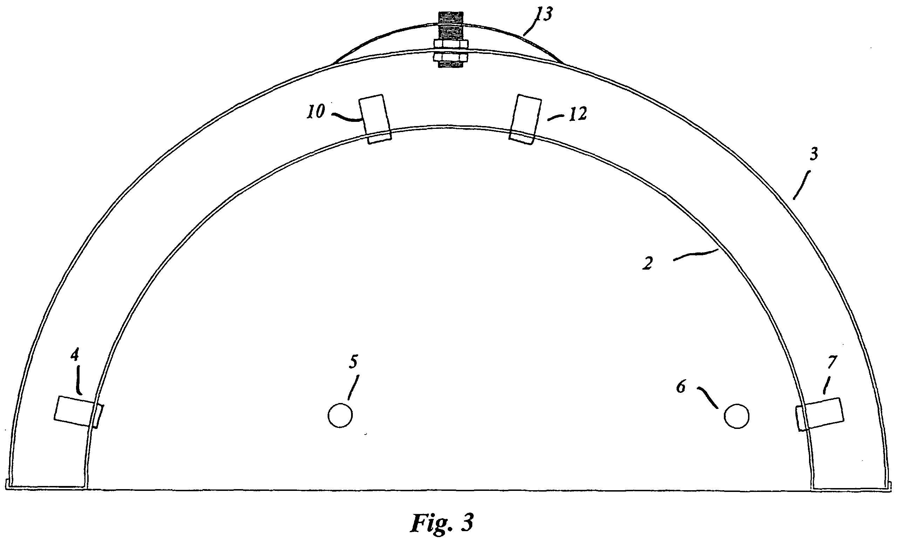

Fig. 3 is a schematic side view of the device of the invention.

With reference now to the above mentioned figures of the drawings, the device 1 for emitting electromagnetic visible waves, comprises a support that preferably has the shape of a first cap 2 in turn contained in a second cap 3 which is preferably connected to a stem like a lamp holder, not shown in the drawings, through a fastening pin centrally arranged on said cap 3.

A plurality of sockets are made on said cap 2, preferably six sockets for receiving a corresponding number of emitters of electromagnetic visible waves, said sockets being arranged substantially on the same plane of the cap 2 along a circumference, said plane being preferably perpendicular to the vertical symmetry axis of the cap.

More particularly the following emitters are provided: a first and a second emitter 4, 7 of orange light in the wave length range between 590 and 630 nm, preferably 625nm; one emitter 5 of red light in the wave length range between 630 and 750nm, preferably 660nm; one emitter 6 of blue light in the wave length range between 430 and 520nm, preferably 470nm; one emitter 8 of green light in the wave length range between 520 and 575nm, preferably 525 nm and one emitter 9 of yellow light in the wave length range between 550 and 600 nm, preferably 580 nm.

The emitters 4, 5, 6, 7, 8, 9 are arranged on said circumference at suitable angular distances, more particularly: having taken as reference said emitter 4, emitters 5 and 9 are angularly spaced to the center of said circumference in opposite directions at an angle between 67 and 77 degrees, preferably 72 degrees; emitter 6 and emitter 8 are angularly spaced from emitters 5 and 9 at an angle between 67 and 77 degrees, preferably 72 degrees, said angles being measured in the same direction of rotation existing between emitters 4 and 5 and emitters 4 and 9 respectively; emitter 7 is angularly spaced from emitters 6 and 8 at an angle between 31 and 41 degrees, preferably 36 degrees, wherein the angles are calculated as above stated respectively.

Moreover each emitter shows on the plane of said circumference a visual angle of the emitted light between 0.5 degrees and 80 degrees, preferably between 20 and 30 degrees.

On the summit of said cap 2 there is another plurality of sockets, preferably three sockets, for a corresponding number of emitters of white light.

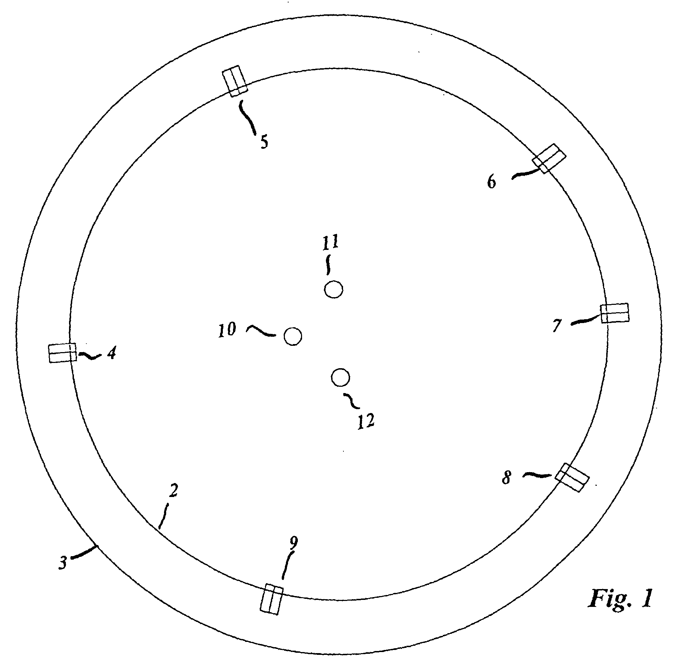

More particularly considering the projection on a horizontal plane shown in Fig. 1, a first emitter 10 of white light relative to the cap top coincident in horizontal projection with the center of said circumference, is arranged in the direction of emitter 4 in such a way that its own projection on the plane of said circumference has a distance from the center of the circumference varying between 5 and 35 mm, preferably 25 mm.

The two other emitters 11 and 12 of white light have their own projections in the plane shown in Fig. 1 on the perpendicular to the segment defined by emitter 4 and the projection of emitter 10, having a distance from the center of said circumference between 5 and 35 mm, preferably 25 mm.

Moreover emitters 10, 11 and 12 on any vertical plane containing them have a visual angle of the emitted light between 0.5 and 80 degrees, preferably between 40 and 60 degrees.

Emitters 4 to 12 are electrically connected to an electric power supply such as for instance a direct current supply substantially consisting of a known transformer not shown in the drawings, fed by the normal voltage of the mains. Said transformer is connected to said emitters for instance through a jack plug to be inserted in a socket arranged on a little cap 13 placed on the summit of cap 3.

Moreover said emitters are connected to electronic means such as a digital timer regulating their on/off times. A cut out switch arranged externally the cap 3 and not shown in the drawings, allows start of the device 1.

For the correct operation of the device 1 of the invention, the device must be put on the head of a person in such a way that the projection of the light emitted from emitter 4 falls between the eyebrows just above the union of the nose.

Pushing said cut out switch, all the emitters are switched on and a corresponding number of light beams of various wave lengths is emitted. After a time interval between 30 and 90 seconds, preferably 60 seconds, the digital timer switches off emitters 10, 11 and 12, switching them on again after a time interval also between 30 and 90 seconds, preferably 60 seconds, and then the cycle is repeated with the same time intervals. When a total time between 30 seconds and 5 minutes, preferably 3 minutes is elapsed, the timer cuts out the device 1.

The entire group of combinations of colors and intermittences generates electromagnetic stimuli to the center of the endocrine system, inducing the epiphysis to redress the altered electric, chemical and mechanical functions without any contact excepting the light beams.

It is to be understood that many modifications, adaptations, integrations, variations and substitutions may be made to the embodiment hereinbefore described as an illustrative and nonlimiting example, however without departing from the scope of the present invention as determined by the appended claims.

## Claims

1. A device for emitting electromagnetic visible waves comprising an external support in the shape of a hemispherical cap to be put on the head of a person, said support being operatively associated to electric and electronic means for setting the switching states of a plurality of emitters of said waves, wherein some emitters (4, 5, 6, 7, 8, 9) are arranged along the circumference on said support in the same plane, while other emitters (10, 11, 12) are arranged near the summit of said support, **characterized in that** the emitters (4, 5, 6, 7, 8, 9) along the circumference comprise a first (4) and a second (7) emitter of orange light in the wave length range between 590 and 630 nm, one emitter (5) of red light in the wave length range between 630 and 750 nm, one emitter (6) of blue light in the wave length range between 430 and 520 nm, one emitter (8) of green light in the wave length range between 520 and 575 nm, and one emitter (9) of yellow light in the wave length range between 550 and 600 nm.

2. The device for emitting electromagnetic visible waves according to claim 1, **characterized in that** said support in the shape of a hemispherical cap to be put on the head of a person comprises a first cap (2) in turn contained in a second cap (3).

3. The device for emitting electromagnetic visible waves according to claim 2, **characterized in that** said first cap (2) is provided with a plurality of sockets for a corresponding number of said emitters (4, 5, 6, 7, 8, 9, 10, 11, 12).

4. The device for emitting electromagnetic visible waves according to claim 3, **characterized in that** six sockets are provided along said circumference of said cap (2) and three sockets are arranged on the summit of said first cap (2) wherein the plane comprising the said emitters (4, 5, 6, 7, 8, 9) being arranged along the circumference is perpendicular to the vertical symmetry axis of said first cap (2).

5. The device according to anyone of the preceding claims wherein the said emitters (10, 11, 12) being arranged near the summit of said support are emitting white light.

6. The device for emitting electromagnetic visible waves according to any one of the preceding claims, **characterized in that** the emitters (4, 5, 6, 7, 8, 9) being arranged along the circumference on said support in the same plane are angularly spaced between each other in such a way that, having taken as reference the first emitter (4) of orange light, emitter (5) of red light and emitter (9) of yellow light are angularly spaced relative to the center of said circumference in opposite directions at an angle between 67 and 77 degrees, emitter (6) of blue light and emitter (8) of green light are angularly spaced from emitter (5) of red light and emitter (9) of yellow light respectively, at an angle between 67 and 77 degrees, and the second emitter (7) of orange light is angularly spaced from emitter (6) of blue light and emitter (8) of green light at an angle between 31 and 41 degrees.

7. The device for emitting electromagnetic visible waves according to anyone of the preceding claim, **characterized in that** each emitter (4, 5, 6, 7, 8, 9) in the plane of said circumference has a visual angle of the emitted light between 0.5 degrees and 80 degrees.

8. The device for emitting electromagnetic visible waves according to claim 5, **characterized in that** the emitters (10, 11, 12) are arranged in such a way that a first emitter (10), arranged near the summit of said first cap (2), which in horizontal projection on the plane of said circumference is coincident with the center of said circumference, is arranged in the direction of the first emitter (4) of orange light in such a way that its own projection on the plane of said circumference has a distance from the center of the circumference varying between 5 and 35 mm, while two other emitters (11, 12) of white light have their own projection on the same plane on the perpendicular to the segment defined by the first emitter (4) of orange light and the projection of said first emitter (10) of white light, at a distance from the center of said circumference between 5 and 35 mm.

9. The device for emitting electromagnetic visible waves according to clam 5, **characterizing in that** said emitters (10, 11, 12) of white light on any vertical plane containing them have a visual angle of the emitted light between 0.5 and 80 degrees.

10. The device for emitting electromagnetic visible waves according to claim 1, **characterizing in that** said electric and electronic means comprise a direct current power supply and a digital timer respectively.

11. The device for emitting electromagnetic visible waves according to claim 10, **characterized in that** said digital timer is set in such a way to provide a plurality of on/off time intervals of at least some emitters connected thereto, said intervals having a duration between 30 and 90 seconds.

## Patentansprüche

1. Vorrichtung zum Emittieren von sichtbaren elektromagnetischen Wellen mit einem externen Träger in der Form einer hemisphärischen Kappe, die auf dem Kopf einer Person zu setzen ist, wobei der Träger funktionsfähig elektrischen und elektronischen Mitteln zum Einstellen der Schaltzustände einer Mehrzahl von Emittern der Wellen zugeordnet ist, wobei einige Emitter (4, 5, 6, 7, 8, 9) entlang dem Umfang auf dem Träger in der gleichen Ebene angeordnet sind, während andere Emitter (10, 11, 12) nahe dem Scheitel des Trägers angeordnet sind, **dadurch gekennzeichnet, dass** die Emitter (4, 5, 6, 7, 8, 9) entlang dem Umfang einen ersten (4) und einen zweiten (7) Emitter von orangefarbenem Licht in dem Wellenlängenbereich zwischen 590 und 630 nm, einen Emitter (5) von rotem Licht in dem Wellenlängenbereich zwischen 630 und 750 nm, einen Emitter (6) von blauem Licht in dem Wellenlängenbereich zwischen 430 und 520 nm, einen Emitter (8) von grünem Licht in dem Wellenlängenbereich zwischen 520 und 575 nm und einen Emitter (9) von gelbem Licht in dem Wellenlängenbereich zwischen 550 und 600 nm umfassen.

2. Vorrichtung zum Emittieren von sichtbaren elektromagnetischen Wellen gemäß Anspruch 1, **dadurch gekennzeichnet, dass** der Träger in der Form einer hemisphärischen Kappe, die auf dem Kopf einer Person zu setzen ist, eine erste Kappe (2) umfasst, die ihrerseits in einer zweiten Kappe (3) enthalten ist.

3. Vorrichtung zum Emittieren von sichtbaren elektromagnetischen Wellen gemäß Anspruch 2, **dadurch gekennzeichnet, dass** die erste Kappe (2) mit einer Mehrzahl von Fassungen für eine entsprechende Anzahl Emitter (4, 5, 6, 7, 8, 9, 10, 11, 12) ausgestattet ist.

4. Vorrichtung zum Emittieren von sichtbaren elektromagnetischen Wellen gemäß Anspruch 3, **dadurch gekennzeichnet, dass** sechs Fassungen entlang des Umfangs der Kappe (2) vorgesehen und drei Fassungen an dem Scheitel der ersten Kappe (2) angeordnet sind, wobei die Ebene mit den entlang des Umfangs angeordneten Emittern (4, 5, 6, 7, 8, 9) senkrecht zu der vertikalen Symmetrieachse der ersten Kappe (2) ist.

5. Vorrichtung gemäß einem der vorhergehenden Ansprüche, bei der die nahe dem Scheitel des Trägers angeordneten Emitter (10, 11, 12) weißes Licht emittieren.

6. Vorrichtung zum Emittieren von sichtbaren elektromagnetischen Wellen gemäß einem der vorhergehenden Ansprüche, **dadurch gekennzeichnet, dass** die Emitter (4, 5, 6, 7, 8, 9), die entlang dem Umfang auf dem Träger in der gleichen Ebene angeordnet sind, winkelmäßig zwischeneinander auf eine solche Art und Weise beabstandet sind, dass, wenn der erste Emitter (4) von orangefarbenem Licht als Bezug genommen wird, der Emitter (5) von rotem Licht und der Emitter (9) von gelbem Licht winkelmäßig bezüglich der Mitte des Umfangs in den gegengesetzten Richtungen mit einem Winkel zwischen 67 und 77 Grad beabstandet sind, der Emitter (6) von blauem Licht und der Emitter (8) von grünem Licht winkelmäßig von dem Emitter (5) von rotem Licht bzw. dem Emitter (9) von gelbem Licht jeweils mit einem Winkel zwischen 67 und 77 Grad beabstandet sind und der zweite Emitter (9) von orangefarbenem Licht winkelmäßig von dem Emitter (6) von blauem Licht und dem Emitter (8) von grünem Licht mit einem Winkel zwischen 31 und 41 Grad beabstandet ist.

7. Vorrichtung zum Emittieren von sichtbaren elektromagnetischen Wellen gemäß einem der vorhergehenden Ansprüche, **dadurch gekennzeichnet, dass** jeder Emitter (4, 5, 6, 7, 8, 9) in der Ebene des Umfangs einen Sehwinkel des emittierten Lichts zwischen 0,5 Grad und 80 Grad aufweist.

8. Vorrichtung zum Emittieren von elektromagnetischen sichtbaren Wellen gemäß Anspruch 5, **dadurch gekennzeichnet, dass** die Emitter (10, 11, 12) auf eine solche Art und Weise angeordnet sind, dass ein erster Emitter (10), der nahe dem Scheitel der ersten Kappe (2) angeordnet ist, die in horizontaler Projektion auf die Ebene des Umfangs mit der Mitte des Umfangs koinzidiert, in der Richtung des ersten Emitters (4) von orangefarbenem Licht auf eine solche Art und Weise angeordnet ist, dass seine eigene Projektion auf die Ebene des Umfangs einen Abstand von der Mitte des Umfangs aufweist, der zwischen 5 und 35 mm variiert, während zwei weitere Emitter (11, 12) von weißem Licht ihre eigene Projektion auf der gleichen Ebene auf der Senkrechten zu dem Segment, das durch den ersten Emitter (4) von orangefarbenem Licht und der Projektion des ersten Emitters (10) von weißem Licht definiert wird, an einem Abstand von der Mitte des Umfangs zwischen 5 und 35 mm aufweisen.

9. Vorrichtung zum Emittieren von sichtbaren elektromagnetischen Wellen gemäß Anspruch 5, **dadurch gekennzeichnet, dass** die Emitter (10, 11, 12) von weißem Licht auf irgendeiner diese enthaltenen vertikalen Ebene einen Sehwinkel des emittierten Licht zwischen 0,5 und 80 Grad aufweisen.

10. Vorrichtung zum Emittieren von sichtbaren elektromagnetischen Wellen gemäß Anspruch 1, **dadurch gekennzeichnet, dass** die elektrischen und elektronischen Mittel eine Gleichstromversorgung bzw. einen digitalen Zeitgeber umfassen.

11. Vorrichtung zum Emittieren von elektromagnetischen sichtbaren Wellen gemäß Anspruch 10, **dadurch gekennzeichnet, dass** der digitale Zeitgeber auf eine solche Art und Weise eingestellt ist, dass eine Mehrzahl von An/Aus-Zeitintervallen von zumindest einigen damit verbundenen Emittern bereitgestellt wird, wobei die Intervalle eine Dauer zwischen 30 und 90 Sekunden aufweisen.

## Revendications

1. Dispositif pour émettre des ondes électromagnétiques visibles comprenant un support externe sous la forme d'un capuchon hémisphérique devant être placé sur la tête d'une personne, ledit support étant associé de façon fonctionnelle à des moyens électriques et électroniques pour régler les états de commutation d'une pluralité d'émetteurs desdites ondes, dans lequel certains émetteurs (4, 5, 6, 7, 8, 9) sont disposés le long de la circonférence sur ledit support dans le même plan, alors que d'autres émetteurs (10, 11, 12) sont disposés près du sommet dudit support, **caractérisé par le fait que** les émetteurs (4, 5, 6, 7, 8, 9) le long de la circonférence comprennent un premier (4) et un second (7) émetteur de lumière orange dans la plage des longueurs d'onde entre 590 et 630 nm, un émetteur (5) de lumière rouge dans la plage des longueurs d'onde entre 630 et 750 nm, un émetteur (6) de lumière bleue dans la plage des longueurs d'onde entre 430 et 520 nm, un émetteur (8) de lumière verte dans la plage des longueurs d'onde entre 520 et 575 nm, et un émetteur (9) de lumière jaune dans la plage des longueurs d'onde entre 550 et 600 nm.

2. Dispositif pour émettre des ondes électromagnétiques visibles, selon la revendication 1, **caractérisé par le fait que** ledit support dans la forme d'un capuchon hémisphérique devant être placé sur la tête d'une personne comprend un premier capuchon (2) contenu à son tour dans un second capuchon (3).

3. Dispositif pour émettre des ondes électromagnétiques visibles, selon la revendication 2, **caractérisé par le fait que** ledit premier capuchon (2) est doté d'une pluralité de douilles pour un nombre correspondant desdits émetteurs (4, 5, 6, 7, 8, 9, 10, 11, 12).

4. Dispositif pour émettre des ondes électromagnétiques visibles, selon la revendication 3, **caractérisé par le fait que** six douilles sont disposées le long de ladite circonférence dudit capuchon (2) et trois douilles sont disposées sur le sommet dudit premier capuchon (2), le plan comprenant lesdits émetteurs (4, 5, 6, 7, 8, 9) qui sont disposés le long de la circonférence étant perpendiculaire à l'axe de symétrie vertical dudit premier capuchon (2).

5. Dispositif selon l'une quelconque des revendications précédentes, dans lequel lesdits émetteurs (10, 11, 12) qui sont disposés près du sommet dudit support sont à émission de lumière blanche.

6. Dispositif pour émettre des ondes électromagnétiques visibles, selon l'une quelconque des revendications précédentes, **caractérisé par le fait que** les émetteurs (4, 5, 6, 7, 8, 9) qui sont disposés le long de la circonférence sur ledit support dans le même plan sont espacés angulairement entre eux d'une manière telle qu'ayant pris comme référence le premier émetteur (4) de lumière orange, l'émetteur (5) de lumière rouge et l'émetteur (9) de lumière jaune sont espacés angulairement par rapport au centre de ladite circonférence dans des directions opposées à un angle entre 67 et 77 degrés, l'émetteur (6) de lumière bleue et l'émetteur (8) de lumière verte sont espacés angulairement respectivement de l'émetteur (5) de lumière rouge et de l'émetteur (9) de lumière jaune, à un angle entre 67 et 77 degrés, et le second émetteur (7) de lumière orange est espacé angulairement de l'émetteur (6) de lumière bleue et de l'émetteur (8) de lumière verte à un angle entre 31 et 41 degrés.

7. Dispositif pour émettre des ondes électromagnétiques visibles, selon l'une quelconque des revendications précédentes, **caractérisé par le fait que** chaque émetteur (4, 5, 6, 7, 8, 9) dans le plan de ladite circonférence a un angle visuel de la lumière émise entre 0,5 degré et 80 degrés.

8. Dispositif pour émettre des ondes électromagnétiques visibles, selon la revendication 5, **caractérisé par le fait que** les émetteurs (10, 11, 12) sont disposés d'une manière telle qu'un premier émetteur (10), disposé près du sommet dudit premier capuchon (2), lequel en projection horizontale sur le plan de ladite circonférence est en coïncidence avec le centre de ladite circonférence, est disposé dans la direction du premier émetteur (4) de lumière orange d'une manière telle que sa propre projection sur le plan de ladite circonférence a une distance du centre de la circonférence variant entre 5 et 35 mm, alors que deux autres émetteurs (11, 12) de lumière blanche ont leur propre projection sur le même plan sur la perpendiculaire au segment défini par le premier émetteur (4) de lumière orange et la projection dudit premier émetteur (10) de lumière blanche, à une distance du centre de ladite circonférence entre 5 et 35 mm.

9. Dispositif pour émettre des ondes électromagnétiques visibles, selon la revendication 5, **caractérisé par le fait que** les émetteurs (10, 11, 12) de lumière blanche sur un quelconque plan vertical les contenant ont un angle visuel de la lumière émise entre 0,5 et 80 degrés.

10. Dispositif pour émettre des ondes électromagnétiques visibles, selon la revendication 1, **caractérisé par le fait que** lesdits moyens électriques et électroniques comprennent respectivement un bloc d'alimentation en courant continu et un registre d'horloge numérique.

11. Dispositif pour émettre des ondes électromagnétiques visibles selon la revendication 10, **caractérisé par le fait que** ledit registre d'horloge numérique est réglé de façon à fournir une pluralité d'intervalles de temps de marche/arrêt d'au moins certains émetteurs connectés à celui-ci, lesdits intervalles ayant une durée entre 30 et 90 secondes.
